# EUROPEAN PATENT SPECIFICATION

(11) **EP 2 516 378 B1**
(45) Date of publication and mention of the grant of the patent: **22.01.2014**
(21) Application number: 10803468.7
(22) Date of filing: 21.12.2010
(51) Int. Cl.: C07C 67/00, C07D 401/04, C07D 401/14, C07F 7/18, C07C 45/51, C07C 49/403, C07C 67/343

(54) **SYNTHESIS OF LIGANDS FOR USE IN ACTINIDE EXTRACTION**
SYNTHESE VON LIGANDEN FÜR ACTINID-EXTRAKTION
SYNTHÈSE DE LIGANDS UTILISABLES POUR L'EXTRACTION D'ACTINIDES

(30) Priority: 23.12.2009 GB 0922588
(43) Date of publication of application: 31.10.2012
(73) Proprietor: The University of Reading, Reading, Berkshire RG6 6AH (GB)
(72) Inventor: LEWIS, Francis, William, Reading Berkshire RG2 8EE (GB); HARWOOD, Laurence, Marius, Woolhampton Reading RG7 5RT (GB); HUDSON, Michael, James, Woodley Reading RG5 4BD (GB)
(74) Representative: Bradley, Adrian
(86) International application number: PCT/GB2010/002305
(87) International publication number: WO 2011/077081

(56) References cited:
- WO-A2-87/03278
- DE-A1- 3 534 613
- US-A- 3 210 404
- P. L. CREGER: J. AM. CHEM. SOC., vol. 89, no. 10, 1967, pages 2500-2501, XP002627429,
- ISHII, AKIHIKO ET AL: "Sulfurization of nonenolizable diketones", JOURNAL OF ORGANIC CHEMISTRY , 55(8), 2421-7 CODEN: JOCEAH; ISSN: 0022-3263, vol. 55, 1990, pages 2421-2427, XP002627430,
- DECKER ET AL: J. MED. CHEM., vol. 52, 27 July 2009 (2009-07-27), pages 7389-7396, XP002627431,
- JONES ET AL: J. MED. CHEM., vol. 41, 1998, pages 3062-3077, XP002627432,
- Aneheim et al: Nuclear Chemistry, Department of Chemical and Biological Engineering, Chalmers University of Technology , 13 March 2009 (2009-03-13), pages 1-62, XP002627639, Retrieved from the Internet: URL:www.skb.se/upload/publications/pdf/R-0 9-03webb.pdf [retrieved on 2011-03-10]
- FOREMAN ET AL: DALTON TRANS., 2006, pages 1645-1653, XP002627433,
- HUDSON ET AL: NEW J. CHEM., vol. 30, 2006, pages 1171-1183, XP002627434,

## Description

The present invention relates to processes for the preparation of 2,2,5,5-tetrasubstitutedhexane-1,6-dicarbonyl compounds. In particular, the invention relates to the synthesis of diethyl 2,2,5,5-tetramethylhexanedioate and dimethyl 2,2,5,5-tetramethylhexanedioate, key intermediates in the synthesis of 3,3,6,6-tetramethylcyclohexane-1,2-dione (CyMe₄-diketone).

### Background to the invention

Certain *N*-heterocyclic ligands are highly selective actinide-extraction agents. Examples of these ligands are abbreviated as CyMe₄-BTBP (1), CyMe₄-BTP (3) and MF₂-BTBP (2).

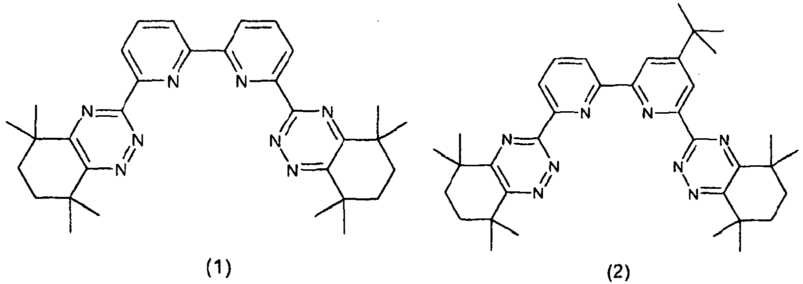

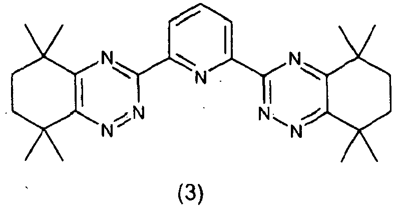

The separation of actinides from lanthanides in high-level liquid waste produced in the nuclear fuel cycle (known as the SANEX process) is envisaged as a key step in the future reprocessing of spent nuclear fuels. Once the actinides are separated and removed from the lanthanides, they may be converted to short-lived radionuclides by neutron fission (transmutation). The remaining waste then loses most of its long-term radiotoxicity, thus reducing the burden on geological disposal. From the numerous European research programmes over the last 30 years (NEWPART, PARTNEW, EUROPART and currently ACSEPT), the ligands shown above have emerged as some of the most promising candidates for use in an industrial SANEX process. In particular, CyMe₄-BTBP (**1**) shows most of the desirable qualities (such as hydrolytic and radiolytic stability, high levels of affinity and selectivity, reversible metal binding which allows stripping, comprises only carbon, hydrogen, oxygen, and nitrogen, is completely incinerable, and has sufficient solubility) for use in an industrial process and, since (**1**) is the most studied and the most well-understood ligand, will almost certainly be used in a future SANEX process. Its suitability for a SANEX process has been demonstrated in a recent 'hot-test' on genuine nuclear waste (Solvent Extr. Ion Exch., 2009, 27, 97). Until now, synthesizing useful and especially large quantities of (**1**) has been problematic owing to the limitations associated with the synthesis of the key intermediate, diketone (**4**).

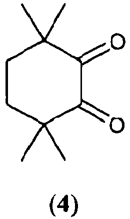

Most known routes to (**4**) involve the cyclisation of dialkyl 2,2,5,5-tetramethylhexanedioate esters (**5**) 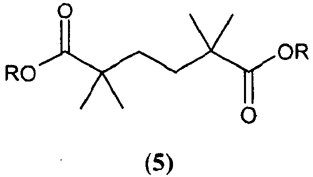 wherein R is methyl or ethyl.

### Brief description of the prior art

Three principal methods have been published in the literature for the synthesis of (**5**):
(a) Dimerization of pivalic acid (6) using Fenton's reagent (hydrogen peroxide and iron (II) sulfate) to afford the diacid (7), followed by esterification to (**5**; **R=Et**) with concentrated sulfuric acid in ethanol is disclosed in J. Am. Chem. Soc., 1958, 80. 2864, Green Chemistry, 2001, 3, 126, Ultrasonics Sonochemistry, 1996, 3, 47, and WO8703278A1. 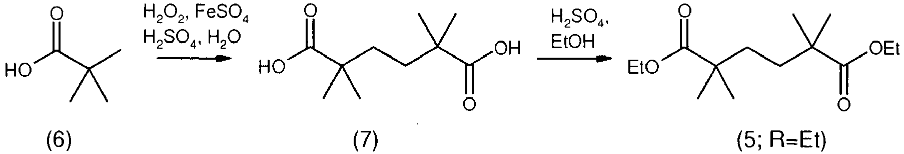
(b) Kolbe electrolysis of the half ester of 2,2-dimethylbutane-1,4-dioic acid (**8**) is disclosed in J. Am. Chem. Soc., 1950, 72, 5388, 1 & EC Product Research and Development, 1964, 3, 105 and Bull. Soc. Chim. Fr., 1988, 3, 571. 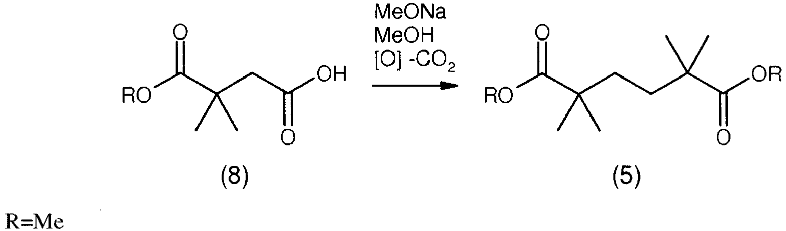
(c) Alkoxycarbonylation of dichloride (**9**) under acidic conditions is disclosed in US 3354198.

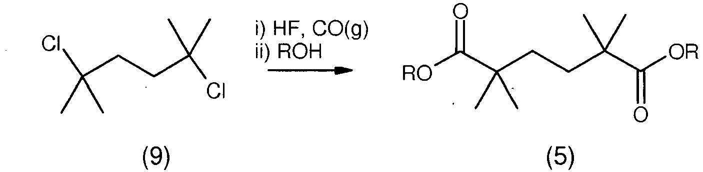

Further, J. Am. Chem. Soc., 1967, 89 (no. 10), 2500-2501 discloses a reaction to make 2,2,5,5-tetramethylhexanedicarboxylic acid (from which (**5**) could be made) from lithiated isobutyric acid and ethylene dichloride, but describes a yield of 0%.

Although the prior art methods do provide processes for the preparation of dimethyl and diethyl 2,2,5,5-tetramethylhexanedioate (**5**), there remains a need for a process which provides these compounds in higher yield. Moreover, there remains a need for a process amenable to the preparation of these compounds on a large scale.

The present invention addresses these and other problems known from the prior art.

### Summary of the Invention

According to a first embodiment, there is provided a process for the preparation of a compound of the formula (**10**) 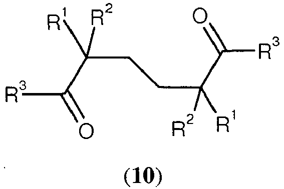
wherein R¹ and R² are independently selected from C₁-C₁₀ alkyl, and
R³ is selected from hydroxy, C₁-C₆ alkyl, C₁-C₆ alkoxy, C₆-C₁₀ aryl, and C₆-C₁₀ aryloxy, or a salt form thereof, comprising reacting a compound of formula (**11**) 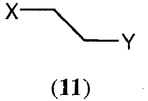
wherein X and Y are independently selected from:
-OSO₂R⁴ wherein R⁴ is selected from C₁-C₁₀ alkyl, C₁-C₁₀ perfluoroalkyl, C₆-C₁₀ aryl, and a 5- or 6-membered unsaturated heterocyclic ring containing one or two heteroatoms selected from the group consisting of nitrogen, oxygen and sulfur; and
-OPOR⁵R⁶, wherein R⁵ and R⁶ are independently selected from C₁-C₁₀ alkyl, C₁-C₁₀ perfluoroalkyl, C₁-C₁₀ alkoxy, C₆-C₁₀ aryl, a 5- or 6-membered unsaturated heterocyclic ring containing one or two heteroatoms selected from the group consisting of nitrogen, oxygen and sulfur, C₆-C₁₀ aryloxy, and heteroaryloxy wherein the heteroaryl group is a 5- or 6-membered unsaturated heterocyclic ring containing one or two heteroatoms selected from the group consisting of nitrogen, oxygen and sulfur;
wherein each aryl or heteroaryl group may be substituted with up to three substituents independently selected from C₁-C₆ alkyl, C₁-C₆ alkoxy, nitro and halogen,
with, in the case where R³ is selected from C₁-C₆ alkyl, C₁-C₆ alkoxy, C₆-C₁₀ aryl, and C₆-C₁₀ aryloxy, a compound of the formula (**12**) 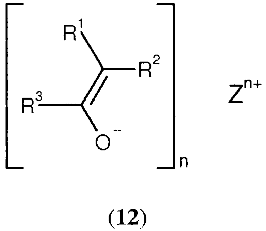
wherein R¹ and R² are as defined above, Z is a cation, and n is 1, 2, 3 or 4; or in the case wherein R³ is hydroxy, a compound of the formula (**12a**) 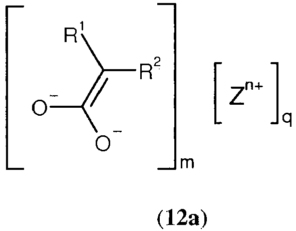
wherein R¹ and R² are as defined above, m, n and q are positive integers such that n×q=2m.

### Detailed description of the preferred embodiments

Preferably, at least one of R¹ and R² is methyl. More preferably, R¹ and R² are both methyl.

Preferably, R³ is C₁-C₆ alkoxy. More preferably, R³ is t-butoxy, methoxy or ethoxy. Most preferably, R³ is ethoxy.

Preferably X and Y are independently selected from -OSO₂R⁴ wherein R⁴ is selected from C₁-C₁₀ alkyl, C₁-C₁₀, perfluoroalkyl, and C₆-C₁₀ aryl optionally substituted with up to three substituents independently selected from C₁-C₆ alkyl, C₁-C₆ alkoxy, nitro and halogen.

More preferably, X and Y are independently selected from p-toluenesulfonale (tosylate or OTs), *p*-bromobenzenesulfonate (brosylate), *p*-nicrobenzenesulfonate (nosylate), methanesulfonate (mesylate) and trifluoromethanesulfonate (triflate or OTf).

More preferably, X and Y are independently selected from trifluoromethanesulfonate or tosylate. Preferably, X and Y are the same.

In a very preferred embodiment X and Y are both trifluoromethanesulfonate. In an alternative very preferred embodiment X and Y are both *p*-toluenesulfonate.

Preferably, Zⁿ⁺ is a cation selected from the group consisting of at least one of alkali metal cations with n=1, alkali earth metal cations with n=2, boron and aluminium cations with n=3, molybdenum, tungsten, manganese, iron, cobalt, nickel, copper, zinc, and unsubstituted or substituted ammonium cations, e.g. the ammonium ion or substituted ammonium cations, especially mono-, di-, tri- or preferably tetrasubstituted ammonium cations where the substitutents are preferably organic moieties bound via a carbon atom and may, for example, be selected from the group consisting of alkyl, such as C₁-C₁₀-alkyl, aryl of 6 to 10 ring atoms. More preferably, Zⁿ⁺ is a cation selected from the group consisting of at least one of alkali metal cations with n=1. Still more preferably, Zⁿ⁺ is a cation selected from Li⁺, Na⁺, K⁺ and Cs⁺. Most preferably, Zⁿ⁺ is Li⁺.

Preferably, compound (**12**) is prepared by reaction of a carbonyl compound of formula (**13**) with a suitable base (**14**) 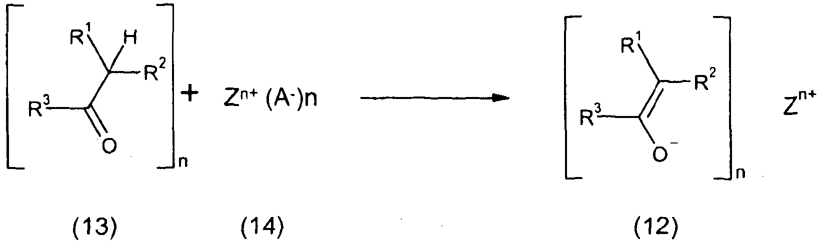 wherein R¹, R² and R³, Z and n are as defined above, and A⁻ is a basic anion. Suitable anions A- include alkoxides, preferably C₁-C₆ alkoxides; alkylamines, preferably C₁-C₆ alkylamines; dialkylamines, preferably di(C₁-C₆)alkylamines, carbanions, preferably C₁-C₆ carbanions; hydride; hydroxide; oxide; carbonate; and silicon-based amides, such as bis(trimethylsilyl)amide. Preferred are di(C₁-C₆)alkylamines, especially diisopropyl amides.

In those embodiments wherein R³ is hydroxy, compound (**12a**) is a dianion, formed by reaction of corresponding carboxylic acid (**13a**) with a suitable base (**14**) 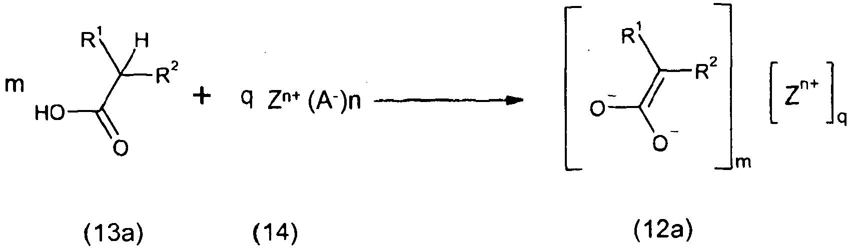 wherein R¹ and R², Z and A⁻ are as defined above, and m, n and q are positive integers such that n×q=2m.

A very preferred base Zⁿ⁺(A⁻)ₙ is lithium diisopropylamide (LDA).

Preferably, at least one molar equivalent (relative to the amount of carbonyl compound (**13**)) of base (**14**) is employed. This helps complete conversion of carbonyl compound to anion (**12**). Preferably, a slight excess of base is used, such as at least 1.05 equivalents, more preferably at least 1.1 equivalents. Preferably, from 1.05 to 1.5, equivalents, more preferably from 1.1 to 1.2 equivalents of base are used.

Reaction of carbonyl compound of formula (**13**) with the base suitably takes place in a solvent. Suitable solvents will be selected by one skilled in the art. Preferred solvents are those which do not react with either base or carbonyl compound. Suitable solvents are selected from alkanes (such as pentane, hexane and octane), aromatic solvents (such as benzene, xylene and toluene), ethers (such as diethyl ether, methyl *t*-butyl ether and diisopropyl ether), cyclic ethers (such as tetrahydrofuran and dioxane), and mixtures of two or more of such solvents. Preferred solvents are ethers, and in particular diethyl ether.

The reaction of the carbonyl compound (**13**) with base (**14**) is suitably conducted at a reduced temperature, such as between 20 and -70 °C, more preferably between 0 and -40 °C, more preferably between -10 and -30 °C, still more.preferably between -15 and -25 °C, most preferably about -20 °C.

Preferably, the reaction of the carbonyl compound (**13**) with base (**14**) is conducted under an inert atmosphere. Suitable inert atmospheres are selected from nitrogen and argon.

The base (**14**) may be added to the carbonyl compound (**13**), or vice versa. The addition of either reagent to the other may be over a suitable period of time, to prevent temperature increases and unwanted side reactions.

Reaction of the base and the carbonyl compound is suitably continued until substantially all the carbonyl compound has been converted to the anion (**12**).

An alternative process for the preparation of (**12**) involves the exchange of cations. In this process, the enolate anion is formed with one specific cation, which is subsequently exchanged for an alternative cation.

Several compounds (**11**) are known *per se* in the art, or are commercially available. Alternatively, these compounds may be prepared using techniques known in the art of organic chemistry. For example, compounds (**11**) wherein X and Y are both selected from -OSO₂R⁴ are suitably prepared from ethylene glycol (**15**) and two equivalents of sulfonyl halide (**16**) or sulfonic anhydride in pyridine or dichloromethane. Dimethylaminopyridine (DMAP) may be used to catalyze the reaction. 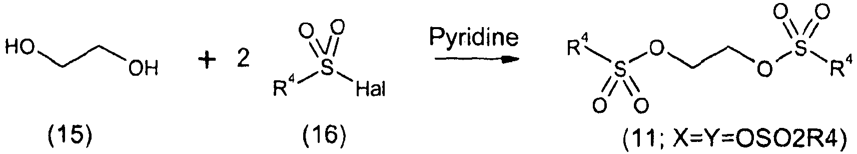 wherein R⁴ is as defined above.

Reaction of enolate (**12**) with (**11**) is preferably conducted in a suitable solvent. Suitable solvents are selected from alkanes (such as pentane, hexane and octane), aromatic solvents (such as benzene, xylene and toluene), ethers (such as diethyl ether, methyl t-butyl ether and diisopropyl ether), cyclic ethers (such as tetrahydrofuran and dioxane), and mixtures of two or more of such solvents. Preferred solvents are ethers, and in particular diethyl ether.

Preferably, compound (**11**) is added to a solution of enolate (**12**). Compound (**11**) may be in solution, in the reaction solvent or another solvent, or may be added in solid form (for example, as aliquots). Preferably, the addition is conducted at a reduced temperature, such as between 20 and -70 °C, more preferably between 0 and -40 °C, more preferably between -10 and -30 °C, still more preferably between -15 and -25 °C, most preferably at about -20 °C.

Preferably, the reaction is conducted under an inert atmosphere, such as a nitrogen or argon atmosphere. Preferably, the reaction is conducted with stirring or agitation. The reaction is preferably heated to achieve an acceptable rate of reaction. Preferably, the reaction is heated at the reflux temperature of the solvent.

The progress of the reaction may be monitored, for example, by using thin layer chromatography, nuclear magnetic resonance spectroscopy, gas chromatography or any other suitable analytical technique.

On completion of the reaction, the desired product is suitably isolated from the reaction mixture by techniques known in the art. Preferably, the mixture is filtered to remove any undissolved solids, the filtrate quenched with weak aqueous acid (such as ammonium chloride), the aqueous and organic phases separated and the organic phase dried over a desiccating agent (such as magnesium sulfate). The solvent is evaporated to leave crude product. Suitable techniques are disclosed in L. M. Harwood and C. J. Moody, Experimental Organic Chemistry: Principles and Practice, Blackwell Science, 1996 and L. M. Harwood, C. J. Moody, J. M. Percy, Experimental Organic Chemistry: Standard and Microscale, Blackwell Science, 1998 (second edition).

The product is purified, if desired, by techniques known to the skilled person, such as recrystallization, distillation, and/or chromatography. Distillation is preferred. Suitable techniques are disclosed in L. M. Harwood and C. J. Moody, Experimental Organic Chemistry: Principles and Practice, Blackwell Science, 1996 and L. M. Harwood, C. J. Moody, J. M. Percy, Experimental Organic Chemistry: Standard and Microscale, Blackwell Science, 1998 (second edition). However, the product need not be purified, and may be suitable for further transformations in crude form.

An advantage of the present route is that the diester compound (**5**) can be produced in larger quantities more rapidly than the above literature routes. This is due to the higher overall yield of (**5**) obtained. In literature route (a) above, the yield quoted for the diacid (**7**) is 37 % and, from the inventor's experience of using this route, the yields are often lower. In the present route, the yield of (**5**; **R=Et**) is 69% when X = OTs and 70 % when X = OTf. The present route also avoids the need to use large electrochemical cells which would place a limit on the reaction scale (route (b) above) or the need to employ high pressures of carbon monoxide and the extremely corrosive hydrofluoric acid which requires the exclusion of glassware (route (c) above).

Another significant advantage of the present route is the minimization of waste and thus costs involved. In particular, the need to use large quantities of pivalic acid and iron (II) sulfate in route (a) above is avoided in the present route. This is particularly significant in the context of large-scale industrial production of (**5**) and thus the diketone (**4**). In addition, the present route also allows for the recycling of the lithium salts produced by filtration of the reaction mixture prior to quenching. Thus, for example, if X = OTs, the lithium tosylate may be recovered and converted back to tosyl chloride, which may then be used to prepare more of the C-2 electrophile ethylene glycol di(p-tosylate) from ethylene glycol, thus closing the production cycle. This represents a further aspect of the invention.

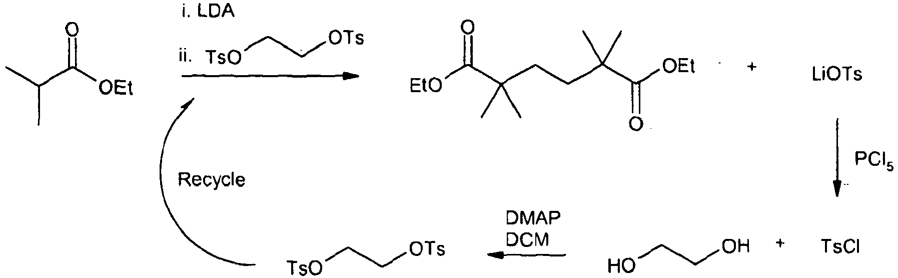

Another significant advantage of the present route is that the diester (**5**), and hence the diketone (4) are obtained in higher purity compared to route (a) above. Thus, in the present route, we have found that the diketone (**4**) is obtained pure without the need for recrystallisation whereas in route (a) above, the crude diketone needs to be recrystallised, thus lowering the yield of (**2**) further (eg: in one typical run, 3.88 g of crude diketone (**2**) obtained from 200 g of pivalic acid in route (a) above was recrystallised to yield 1.05 g of pure diketone (**4**); overall yield of diketone (**4**) = < 1 %. This compares with an overall yield of the diketone (**4**) = 33 % (X = OTs) or 41 % (X = OTf) when using the present method.

In a further embodiment, the invention comprises the further steps of converting a compound of formula (**10**) wherein R³ is C₁-C₆ alkoxy to 3,3,6,6-tetrasubstitutedcyclohexane-1,2-dione (17). This conversion is achieved using methods known in the art.

A first method involves conducting an intramolecular acyloin reaction of a compound of formula (**10**) wherein R³ is C₁-C₆ alkoxy in the presence of chlorotrimethylsilane to give 1,2-bis-(siloxene) (**18**). This compound is subsequently oxidized, e.g. with bromine in carbon tetrachloride to give diketone (**17**). The inventors have also discovered that carbon tetrachloride may be replaced with dichloromethane with no adverse consequences in terms of yield. This change of solvent has significant advantages in terms of safety, availability of solvent and environmental profile, and represents a further aspect of the invention. 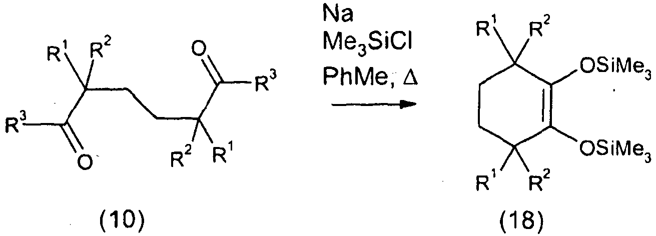 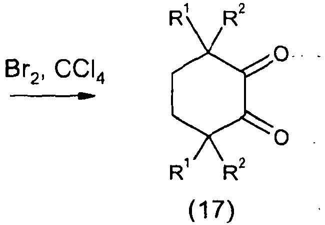
R³ = C₁-C₆ alkoxy; R¹, R² as defined above.

A second method involves conducting an intramolecular acyloin reaction to give hydroxyketone (**19**). This compound is subsequently oxidized e.g. with chromium (VI) oxide or thionyl chloride to give diketone (**17**). 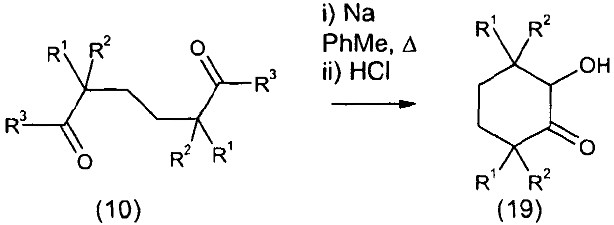 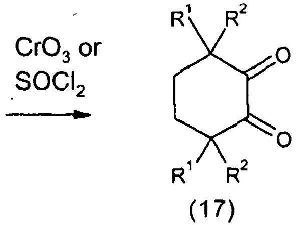
wherein R¹ and R² are as defined above.

In a further embodiment, the invention comprises the further steps of converting a 3,3,6,6-tetrasubstitutedcyclohexane-1,2-dione (**17**) into 6,6'-Bis(5,5,8,8-tetrasubstituted-5,6,7,8-tetrahydro-1,2,4-benzotriazin-3-yl)-2,2'-bipyridine (**20**), and the 4-*t*-butyl analogue (**21**), comprising reacting 2,2'-bipyridine-6,6'-dicarbohydrazonamides (22) or (**23**) with 3,3,6,6-tetrasubstitutedcyclohexane-1,2-dione (**17**) in the presence of a base such as triethylamine. In the hands of the inventors, the use of THF gave a yield of (20) of only 10 %, whereas the reported yield of (20) using this solvent is 60 % (see M. R. S. Foreman, M. J. Hudson, M. G. B. Drew, C. Hill, C. Madic, Dalton Trans., 2006, 1645). Surprisingly, the use of dioxane as a solvent has been found to give a yield of (**20**) of 56 %. The use of dioxane in this context represents a further aspect of the invention.

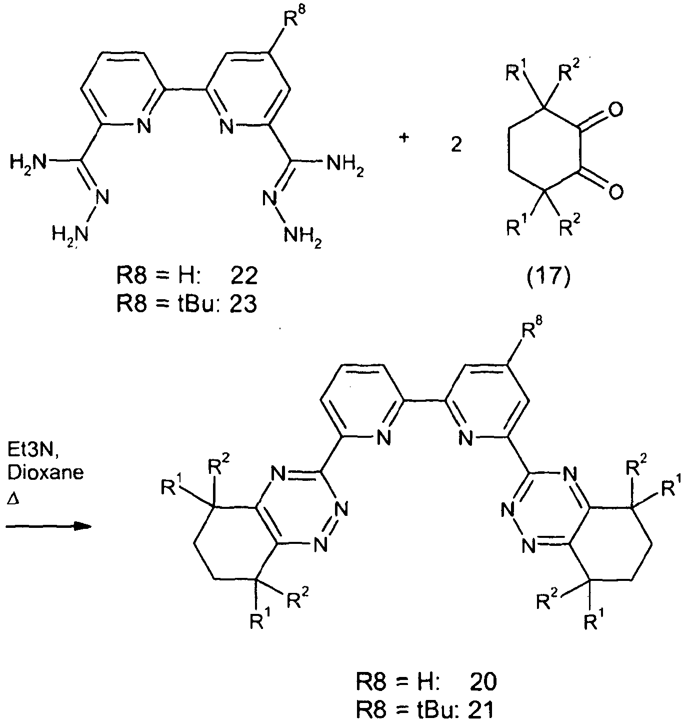

In a further embodiment, the invention comprises the further steps of converting a 3,3,6,6-tetrasubstituted cyclohexane-1,2-dione (**17**) into 2.6-bis(5,5,8,8-tetrasubstituted-5,6,7,8-tetrahydro-1,2,4-benzotriazin-3-yl)-pyridine (**24**), comprising reacting pyridine-2,6-dicarbohydrazonamide (**25**) with 3,3,6;6-tetrasubstitutedcyclohexane-1,2-dione (**17**) in the presence of a base, such as triethylamine. Conditions for performing this transformation are disclosed in New J. Chem.. 2006, 30, 1171.

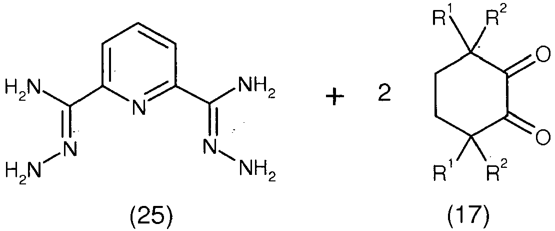

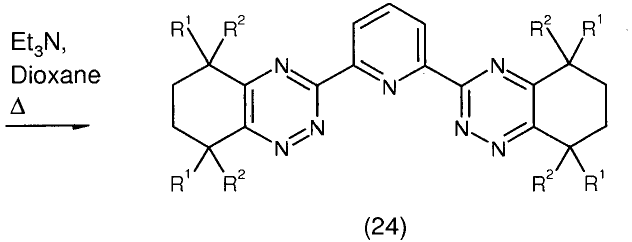

In a further embodiment, the invention comprises the further steps of converting a 3,3,6,6-tetrasubstitutedcyclohexane-1,2-dione (**17**) to a compound of formula (**25**)

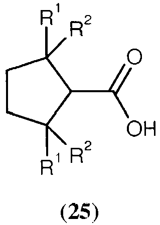

Suitable techniques for effecting this transformation are disclosed for example in WO8703278.

Diketone (**4**) may be used to synthesize quinoxaline heterocyclic compounds of general structure (**27**). These compounds exhibit retinoid activity and act as agonists/antagonists of Retinoic acid, which is the oxidised form of Vitamin A and is used as a drug in the treatment of various dermatological and inflammatory conditions (eg: rheumatoid arthritis, colitis, psoriasis and acne vulgaris) as well as leukaemia. Methods for achieving the conversion are set out in WO9613478 and WO9702244.

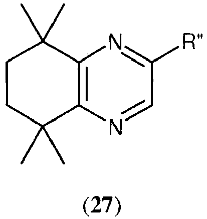

### Definitions

### Alkyl

Alkyl, as used herein refers to an aliphatic hydrocarbon chain and includes straight and branched chains e. g. of 1 to 6 carbon atoms such as methyl, ethyl, n-propyl, isopropyl, n-butyl, isobutyl, sec-butyl, t-butyl, n-pentyl, isopentyl, neo-pentyl, n-hexyl, and isohexyl.

### Alkoxy

Alkoxy as used herein refers to the group -O-alkyl, wherein alkyl is as defined above. Examples of alkoxy groups include methoxy, ethoxy, n-propoxy, isopropoxy, n-butoxy, isobutoxy. sec-butoxy, t-butoxy, n-pentoxy, isopentoxy, neo-pentoxy, n-hexyloxy, and isohexyloxy.

### Perfluoroalkyl

As used herein, the term "perfluoroalkyl" refers to an alkyl group as hereinbefore defined, wherein all the hydrogen atoms have been replaced with fluorine atoms.

Examples of perfluoroalkyl groups are trifluoromethyl, pentafluoroethyl, and nonafluorobutyl.

### Perfluoroalkoxy

As used herein, the term "perfluoroalkoxy" refers to an alkoxy group as hereinbefore defined, wherein all the hydrogen atoms have been replaced with fluorine atoms. Examples of perfluoroalkyl groups are trifluoromethoxy, pentafluoroethoxy, and nonafluorobutoxy.

### Aryl

As used herein, "aryl" refers to an unsaturated aromatic carbocyclic group of from 6 to 10 carbon atoms having a single ring (e. g., phenyl) or multiple condensed (fused) rings (e.g., naphthyl). Preferred aryl groups include phenyl, naphthyl and the like.

### Heteroaryl

As used herein, the term "heteroaryl" refers to a 5- or 6-membered unsaturated heterocyclic ring containing one or two heteroatoms selected from the group consisting of nitrogen, oxygen and sulfur. Examples of heteroaryl groups include thiophene, furan, pyridine, pyrimidine, pyridazine, imidazole, isoxazole, oxadiazoles, quinolines, benzotriazines and the like.

### Aryloxy

As used herein, "aryloxy" refers to the group -O-aryl, wherein aryl is as defined above. Preferred aryloxy groups include phenoxy, naphthyloxy and the like.

### Heteroaryloxy

As used herein, "heteroaryloxy" refers to the group -O-heteroaryl, wherein heteroaryl is as defined above.

### Leaving Group

The term "leaving group" as used herein, refers to a group capable of being displaced from a molecule when said molecule undergoes reaction with a nucleophile.

### Salt form

As used herein, the term "salt form" includes salts of alkali metals, including lithium, potassium and caesium, alkaline earth metals, including magnesium, calcium and strontium, ammonium salts, such as ammonium and alkylammonium salts.

### Examples:

### General:

Anhydrous diethyl ether was dried and distilled from sodium benzophenone ketyl immediately prior to use. Diisopropylamine was dried and distilled over calcium hydride immediately prior to use. Toluene was dried over calcium-chloride prior to use. All other reagents were obtained from Aldrich Chemical Company Inc.

### Diethyl 2,2,5,5-tetramethythexanedioate (5; R=Et)

### Method A:

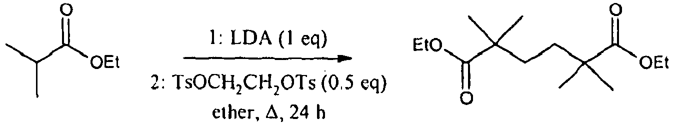

Anhydrous diethyl ether (500 mL) was placed in an oven-dried 1L 3-neck flask and sealed under an atmosphere of nitrogen. Diisopropylamine (40.36 mL, 1.1 equiv) was added via syringe and the solution was cooled to -20 °C using a dry ice-acetone bath. *n-*Butyllithium (104.73 mL, 2.5 M, 1 equiv) was added dropwise via syringe and the solution was stirred at - 20 °C for 1h. Ethyl isobutyrate (35 mL, 30.42 g. 261.837 mmol) was slowly added dropwise via syringe over 45 mins and the solution was then allowed to warm to room temperature and stirred for an additional 1h. Solid ethylene di(p-toluenesulfonate) (48.49 g. 0.5 equiv) was added in small aliquots over 10 mins and the suspension was heated under reflux for 24 h. The flask was allowed to cool to room temperature and the insoluble solid was filtered and washed with ether (100 mL) and DCM (200 mL). The resulting white solid (43.8 g, 94 %) was shown by ¹H NMR to be pure lithium *p*-toluenesulfonate. The filtrate was quenched with satd. aq. ammonium chloride (200 mL) and the phases were mixed and separated. The aqueous phase was extracted with ether (100 mL). The combined organic extracts were washed with water (150 mL), dried over anhydrous magnesium sulfate and evaporated under reduced pressure to afford the crude product (38.2) g) as a yellow liquid. The crude product was purified by vacuum distillation using a 10 inch tall Vigreux colunm to afford the title compound (23.35 g, 69 %) as a clear liquid (Bp 72-76°C at 13.3 Pa (0.1 mm Hg)). Two additional fractions were obtained (3.02 g, Bp 52-60 °C and 2.86 g, Bp 98-104 °C) which were shown by ¹H NMR to be unidentified impurities and unreacted ethylene di(p-toluenesulfonate), respectively.

### Method B:

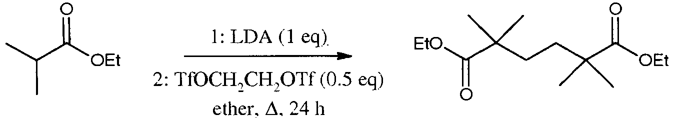

Anhydrous diethyl ether (100 mL) was placed in an oven-dried 250 mL 3-neck flask and sealed under an atmosphere of nitrogen. Diisopropylamine (4.86 mL, 1.1 equiv) was added via syringe and the solution was cooled to - 20 °C using a dry ice-acetone bath. *n*-Butyllithium (19.7 mL, 1.6 M, I equiv) was added dropwise via syringe and the solution was stirred at - 20 °C for 1 h. Ethyl isobutyrate (4.21 mL, 3.66 g, 31.533 mmol) was slowly added dropwise via syringe over 30 mins and the solution was then allowed to warm to 0 °C and stirred for an additional 1 h. A solution of ethylene bis(trifluoromethanesulfonate) (5.14 g, 0.5 equiv) in anhydrous diethyl ether (15 mL) was added dropwise via syringe over 30 mins and the solution was allowed to warm to room temperature and stirred for 1 h. The solution was then heated under reflux for 24 h.

The flask was allowed to cool to room temperature, the solution was quenched with satd. aq. ammonium chloride (50 mL) and the phases were mixed and separated. The aqueous phase was extracted with ether (50 mL). The combined organic extracts were washed with water (50 mL), dried over anhydrous magnesium sulfate and evaporated under reduced pressure to afford the crude product (4.39 g) as a yellow liquid. The crude product was purified by vacuum distillation using a 6 inch tall Vigreux column to afford the title compound (2.85 g, 70 %) as a clear liquid (Bp 72-76 °C at 13.3 Pa (0.1mm Hg)). One additional fraction was obtained (0.08 g, Bp 58-62 °C) which was shown by ¹H NMR to be unidentified impurities.
¹H NMR (400 MHz, CDCl₃): δ 1.15 (12 H, s, 2 × 2-C*H*₃ and 2 × 5-C*H*₃), 1.25. (6H, t. *J* 7.8 Hz, CH₂C*H*₃), 1.45 (4H, s, 3-C*H*₂ and 4-C*H*₂), 4.12 (4H, q, *J* 7.8 Hz, C*H*₂CH₃) ppm.

### 1,2-Bis(trimethylsilyloxy)-3,3,6,6-tetramethylcyclohex-1-ene

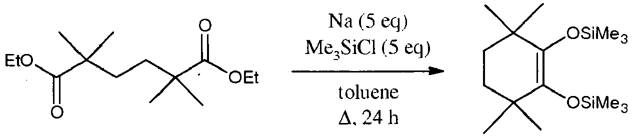

Anhydrous toluene (300 mL) was placed in an oven-dried 500 mL 1-neck flask and sealed under an atmosphere of nitrogen. Sodium (10.41 g, 5 equiv) was added and the flask was heated under reflux until the sodium melted. The starting material (23.35 g, 90.503 mmol) was added and then chlorotrimethylsilane (57.20 mL, 5 equiv) was added. The mixture was heated under reflux for 24 h. The mixture was allowed to cool to room temperature and was suction-filtered through a sintered disk under nitrogen using a wide Schlenk tube. The solid residue was washed with toluene (100 mL) and THF (50 mL) and the filtrate was evaporated under reduced pressure to afford the crude product (26.57 g) which was purified by vacuum distillation to afford the title compound (17.79 g, 63 %) as a clear liquid (Bp 68-72 °C at 13.3 Pa (0.1mm Hg)). Two additional fractions were obtained (1.23 g, Bp 48-60 °C and 1.43 g, Bp 82-86 "C) which were shown by ¹H NMR to be impure product and unidentified impurities. The excess sodium was quenched under nitrogen by washing the solid residue with EtOH (150 mL).
¹H NMR (400 MHz, CDCl₃): δ 0.00 (18 H, s, 2 × OSi(C*H*₃)₃), 0.83 (12 H, s, 2 × 3-C*H*₃ and 2 × 6-C*H*₃), 1.25 (4H, s, 4-C*H*₂ and 5-C*H*₂) ppm.

### 3,3,6,6-Tetramethylcyclohexane-1,2-dione (4)

### Method A:

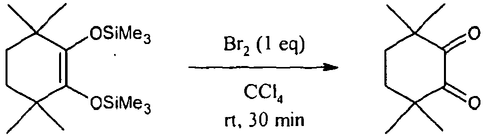

The starting material (20.14 g, 64.166 mmol) was dissolved in carbon tetrachloride (130 mL) in a 250 mL 1-neck flask. Bromine (3.28 mL, 1 equiv) was added dropwise over 15 mins. The solution was stirred at room temperature for 30 mins. The solution was then washed with water (2 × 75 mL) and satd. aq. sodium sulfite (50 mL), dried over magnesium sulfate and evaporated under reduced pressure to afford the title compound as a yellow solid (10.68 g, 99 %).

### Method B:

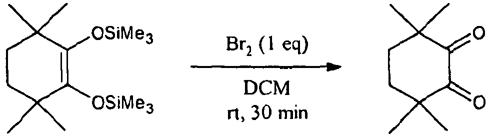

The starting material (1.78 g, 5.668 mmol) was dissolved in DCM (20 mL) in a 100 mL 1-neck flask. Bromine (0.29 mL, 1 equiv) was added dropwise over 5 mins. The solution was stirred at room temperature for 30 mins. The solution was diluted with DCM (50 mL) and then washed with water (2 × 20 mL) and satd. aq. sodium sulfite (30 mL), dried over magnesium sulfate and evaporated under reduced pressure to afford the title compound as a yellow solid (0.94 g, 99 %).
¹H NMR (400 MHz, CDCl₃): δ 1.15 (12 H, s, 2 × 3-C*H*₃ and 2 × 6-C*H*₃), 1.87 (4H, s, 4-C*H*₂ and 5-C*H*₂) ppm.

### 6,6'-Bis(5,5,8,8-tetramethyl-5,6,7,8-tetrahydro-1,2,4-benzotriazin -3-yl)-2,2'-bipyridine

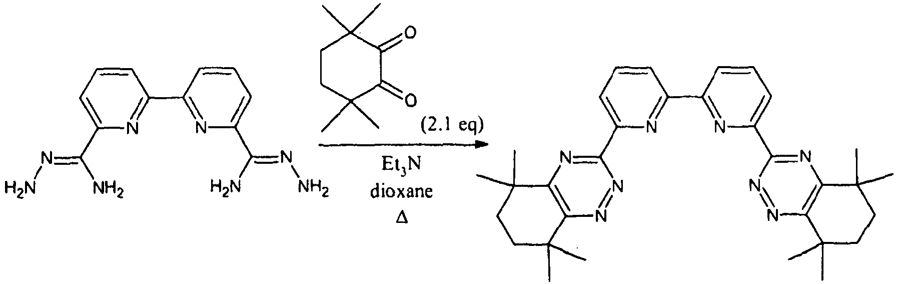

2,2'-Bipyridine-6,6'-dicarbohydrazonamide (2.51 g, 9.296 mmol) was suspended in dioxane (300 mL) and 3,3,6,6-tetramethylcyclohexane-1,2-dione (3.28 g, 2.1 equiv) was added. Triethylamine (25 mL) was added and the flask was heated under reflux for 24 h. The flask was allowed to cool to room temperature, the mixture was filtered and the insoluble solid was washed with THF (50 mL). The filtrate was evaporated under reduced pressure to afford the crude product as an orange solid (5.74 g). The crude product was triturated with EtOH (100 mL) and the insoluble solid was filtered and washed with EtOH (50 mL) and diethyl ether (40 mL) to afford the title compound as a yellow solid (1.31 g). The filtrate was evaporated under reduced pressure and the resulting solid was again triturated with EtOH (150 mL) and the insoluble solid was filtered and washed with EtOH (50 mL) and diethyl ether (20 mL) to afford an additional 1.46 g of product. Total yield: 2.77 g (56 %).
¹H NMR (400 MHz, CDCl₃): δ 1.48 (12 H, s, 4 × 5-C*H*₃), 1.53 (12 H, s, 4 × 8-C*H*₃), 1.90 (8H, s, 2 × 6-C*H*₂ and 2 × 7-CH₂), 8.04 (2H, t, *J* 7.8 Hz, 4-C*H* and 4'-C*H*), 8.54 (2H, dd, *J* 7.8 and 0.8 Hz, 5-C*H* and 5'-CH), 8.96 (2H, dd, *J* 7.8 and 0.8 Hz, 3-C*H* and 3'-C*H*) ppm.
¹³C NMR (100 MHz, CDCl₃): δ 29.2 (4 × 5-CH₃), 29.7 (4 × 8-CH₃), 33.3 (2 × 6-CH₂), 33.8 (2 × 7-CH₂), 36.5 (2 × quat), 37.2 (2 × quat), 122.8 (C-3 and C-3'), 123.9 (C-5 and C-5'), 137.8 (C-4 and C-4'), 152.8 (2 × quat), 156.1 (2 × quat), 160.9 (2 × quat), 163.0 (2 × quat), 164.3 (2 × quat) ppm.

## Claims

1. A process for the preparation of a compound of the formula (**10**) 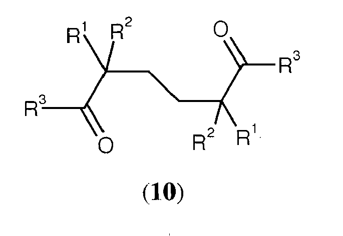
wherein R¹ and R² are independently selected from C₁-C₁₀ alkyl, and
R³ is selected from hydroxy, C₁-C₆ alkyl, C₁-C₆ alkoxy, C₆-C₁₀ aryl, and C₆-C₁₀ aryloxy or a salt form thereof, comprising
reacting a compound of formula (**11**) 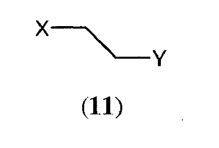
wherein X and Y are independently selected from:
-OSO₂R⁴ wherein R⁴ is selected from C₁-C₁₀ alkyl, C₁-C₁₀ perfluoroalkyl, C₆-C₁₀ aryl, and a 5- or 6-membered unsaturated heterocyclic ring containing one or two heteroatoms selected from the group consisting of nitrogen, oxygen and sulfur; and
-OPOR⁵R⁶, wherein R⁵ and R⁶ are independently selected from C₁-C₁₀ alkyl, C₁-C₁₀ perfluoroalkyl, C₁-C₁₀ alkoxy, C₆-C₁₀ aryl, a 5- or 6-membered unsaturated heterocyclic ring containing one or two heteroatoms selected from the group consisting of nitrogen, oxygen and sulfur, C₆-C₁₀ aryloxy, and heteroaryloxy wherein the heteroaryl group is a 5- or 6-membered unsaturated heterocyclic ring containing one or two heteroatoms selected from the group consisting of nitrogen, oxygen and sulfur;
wherein each aryl or heteroaryl group may be substituted with up to three substituents independently selected from C₁-C₆ alkyl, C₁-C₆ alkoxy, nitro and halogen,
with, in the case where R³ is selected from C₁-C₆ alkyl, C₁-C₆ alkoxy, C₆-C₁₀ aryl, and C₆-C₁₀ aryloxy, a compound of the formula (**12**) 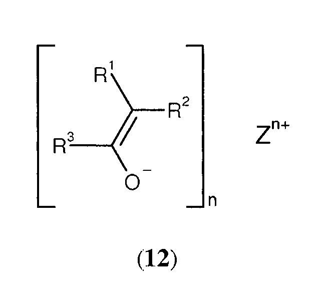
wherein R¹ and R² are as defined above, Z is a cation, and n is 1, 2, 3 or 4; or in the case wherein R³ is hydroxy, a compound of the formula (**12a**) 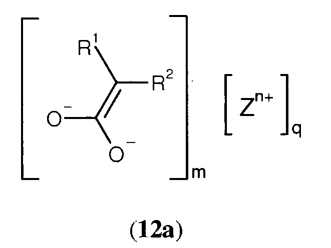
wherein R¹ and R² are as defined above, m, n and q are positive integers such that n×q=2m.

2. A process according to claim 1 wherein R¹ and R² are both methyl.

3. A process according to claim 1 or 2 wherein R³ is C₁-C₆ alkoxy.

4. A process according to claim 3 wherein R³ is methoxy or ethoxy.

5. A process according to claim 1 wherein X and Y are both trifluoromethanesulfonate or tosylate.

6. A process according to any preceding claim wherein Zⁿ⁺ is Li⁺.

7. A process according to any preceding claim comprising the further step of converting the compound of formula (10) wherein R₃ is selected from C₁-C₆ alkoxy to a bis(trimethylsilyloxy) cyclohex-1-ene of formula (**18**) 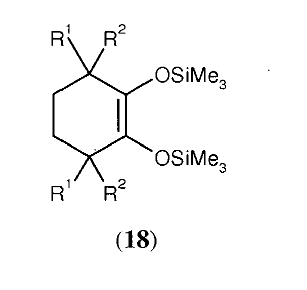
wherein R¹ and R² are as defined in claim 1.

8. A process according to any preceding claim, comprising the further step or steps of converting the compound of formula (**10**) or (**18**) to cyclohexane-1,2-dione (**17**) 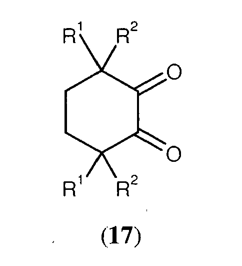
wherein R¹ and R²are as defined in claim 1.

9. A process according to any preceding claim, comprising the further step or steps of converting the compound of formula (**10**), (**17**) or (**18**) to give a compound of formula (**20**), (**21**), (**24**) or (**26**) 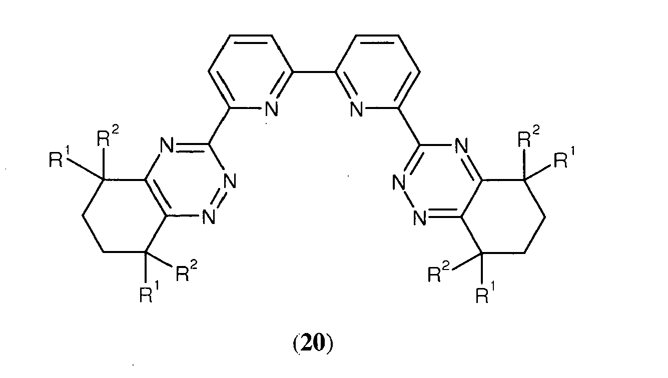 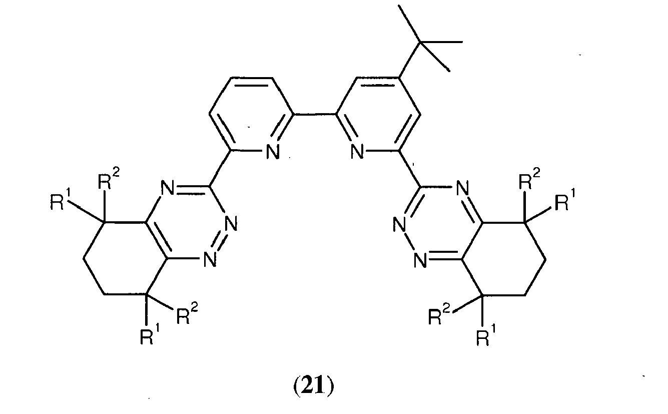 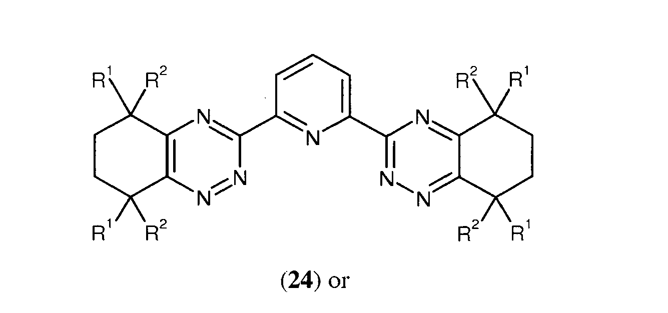 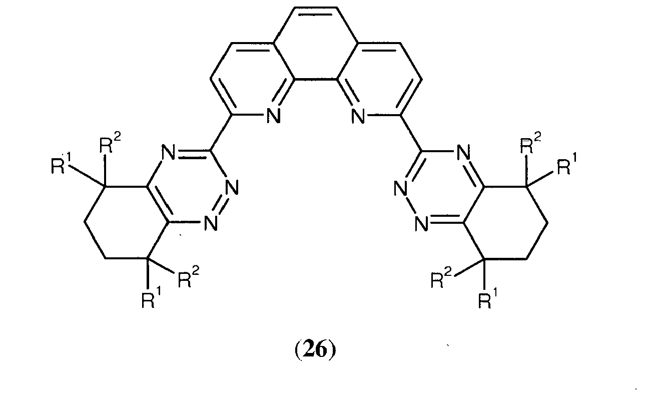
wherein R¹ and R² are as defined in claim 1.

10. A process according to any preceding claim, comprising the further step or steps of converting the compound of formula (**10**), (**17**) or (**18**) to a compound of formula (**25**) 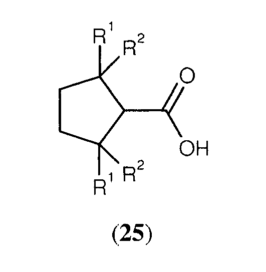
wherein R¹ and R² are as defined in claim 1.

## Patentansprüche

1. Verfahren zur Herstellung einer Verbindung der Formel (10) 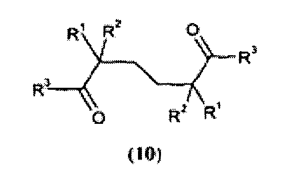
worin R¹ und R² unabhängig ausgewählt sind aus C₁-C₁₀ Alkyl und
R³ ausgewählt ist aus Hydroxy, C₁-C₆ Alkyl, C₁-C₆ Alkoxy, C₆-C₁₀ Aryl und C₆-C₁₀ Aryloxy oder ein Salz davon, umfassend
das Umsetzen einer Verbindung der Formel (11) 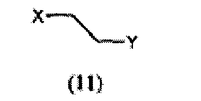
worin X und Y unabhängig ausgewählt sind aus:
-OSO₂R⁴, wobei R⁴ ausgewählt ist aus C₁-C₁₀ Alkyl, C₁-C₁₀ Perfluoralkyl, C₆-C₁₀ Aryl und einem 5- oder 6-gliedrigen ungesättigten heterocyclischen Ring, der ein oder zwei Heteroatome enthält, ausgewählt aus der Gruppe, bestehend aus Stickstoff, Sauerstoff und Schwefel; und
-OPOR⁵R⁶, wobei R⁵ und R⁶ unabhängig ausgewählt sind aus C₁-C₁₀ Alkyl, C₁-C₁₀ Perfluoralkyl, C₁-C₁₀ Alkoxy, C₆-C₁₀Aryl, einem 5- oder 6-gliedrigen ungesättigten heterocyclischen Ring, der ein oder zwei Heteroatome enthält,
ausgewählt aus der Gruppe, bestehend aus Stickstoff, Sauerstoff und Schwefel, C₆-C₁₀ Aryloxy und Heteroaryloxy, wobei die Heteroarylgruppe ein 5- oder 6-gliedriger ungesättigter heterocyclischer Ring ist, der ein oder zwei Heteroatome enthält, ausgewählt aus der Gruppe, bestehend aus Stickstoff, Sauerstoff und Schwefel;
wobei jede Aryl- oder Heteroarylgruppe mit bis zu drei Substituenten substituiert sein kann, die unabhängig ausgewählt sind aus C₁-C₆ Alkyl, C₁-C₆ Alkoxy, Nitro und Halogen,
mit, in dem Fall, in dem R³ ausgewählt ist aus C₁-C₆ Alkyl, C₁-C₆ Alkoxy, C₆-C₁₀ Aryl und C₆-C₁₀Aryloxy, einer Verbindung der Formel (12) 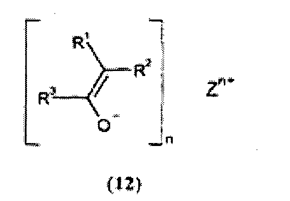
worin R¹ und R² wie oben definiert sind, Z für ein Kation steht und n 1, 2, 3 oder 4 ist; oder
in dem Fall, in dem R³ Hydroxy ist, einer Verbindung der Formel (12a) 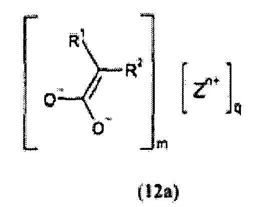
worin R¹ und R² wie oben definiert sind, m, n und q positive ganze Zahlen sind, so dass nxq=2m ist.

2. Verfahren nach Anspruch 1, worin R¹ und R² Methyl sind.

3. Verfahren nach Anspruch 1 oder 2, worin R³ C₁-C₆ Alkoxy ist.

4. Verfahren nach Anspruch 3, worin R³ Methoxy oder Ethoxy ist.

5. Verfahren nach Anspruch 1, worin X und Y Trifluormethansulfonat oder Tosylat sind.

6. Verfahren nach einem der vorangegangenen Ansprüche, wobei Zn⁺ für Li⁺ steht.

7. Verfahren nach einem der vorangegangen Ansprüche, umfassend einen weiteren Schritt des Umwandelns der Verbindung (10), worin R³ ausgewählt ist aus C₁-C₆ Alkoxy, in ein bis(Trimethylsilyloxy)cyclohex-1-en der Formel (18) 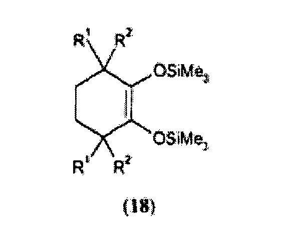
worin R¹ und R² wie in Anspruch 1 definiert sind.

8. Verfahren nach einem der vorangegangen Ansprüche, umfassend einen weiteren Schritt oder Schritte des Umwandelns der Verbindung der Formel (10) oder (18) in Cyclohexan-1,2-dion (17) 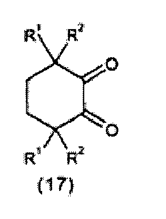
worin R¹ und R² wie in Anspruch 1 definiert sind.

9. Verfahren nach einem der vorangegangen Ansprüche, umfassend einen weiteren Schritt oder Schritte des Umwandelns der Verbindung der Formel (10), (17) oder (18), um eine Verbindung der Formel (20), (21), (24) oder (26) zu erhalten 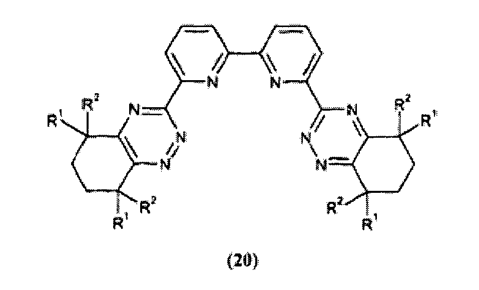 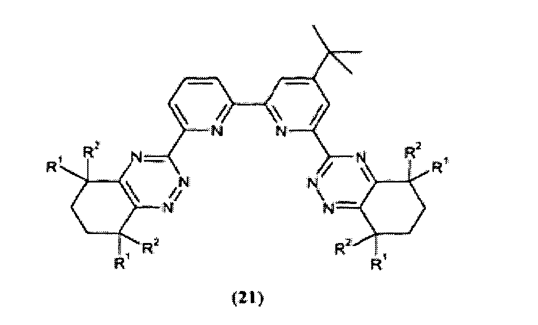 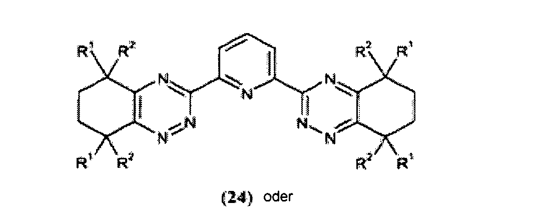 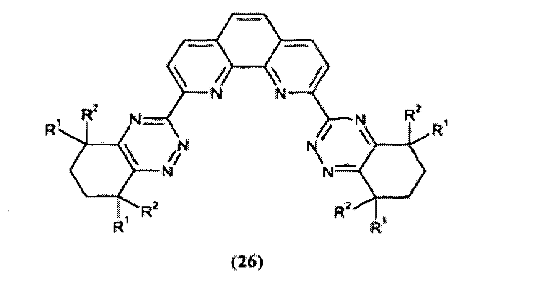
worin R¹ und R² wie in Anspruch 1 definiert sind.

10. Verfahren nach einem der vorangegangen Ansprüche, umfassend einen weiteren Schritt oder Schritte des Umwandelns der Verbindung der Formel (10), (17) oder (18) in eine Verbindung der Formel (25) 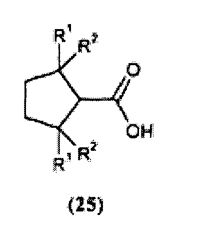
worin R¹ und R² wie in Anspruch 1 definiert sind.

## Revendications

1. Procédé pour la préparation d'un composé de formule (10) 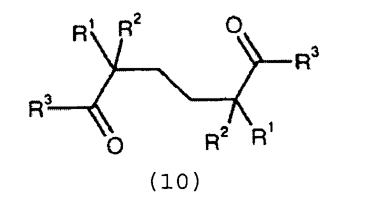
dans laquelle R¹ et R² sont indépendamment choisis parmi les radicaux alkyle en C₁ à C₁₀ et R³ est choisi parmi les radicaux hydroxy, alkyle en C₁ à C₆, alcoxy en C₁ à C₆, aryle en C₆ à C₁₀, et aryloxy en C₆ à C₁₀,
ou d'une forme sel de celui-ci,
comprenant
la réaction d'un composé de formule (11) 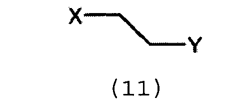
dans laquelle X et Y sont indépendamment choisis parmi :
-OSO₂R⁴ où R⁴ est choisi parmi les radicaux alkyle en C₁ à C₁₀, perfluoroalkyle en C₁ à C₁₀, aryle en C₆ à C₁₀, et les hétérocycles insaturés à 5 ou 6 chaînons contenant un ou deux hétéroatomes choisis dans le groupe constitué par l'azote, l'oxygène et le soufre ; et
-OPOR⁵R⁶ où R⁵ et R⁶ sont indépendamment choisis parmi les radicaux alkyle en C₁ à C₁₀, perfluoroalkyle en C₁ à C₁₀, alcoxy en C₁ à C₁₀, aryle en C₆ à C₁₀, les hétérocycles insaturés à 5 ou 6 chaînons contenant un ou deux hétéroatomes choisis dans le groupe constitué par l'azote, l'oxygène et le soufre, aryloxy en C₆ à C₁₀, et hétéroaryloxy, où le groupe hétéroaryle est un hétérocycle insaturé à 5 ou 6 chaînons contenant un ou deux hétéroatomes choisis dans le groupe constitué par l'azote, l'oxygène et le soufre ;
où chaque groupe aryle ou hétéroaryle peut être substitué par jusqu'à trois substituants indépendamment choisis parmi les halogènes et les radicaux alkyle en C₁ à C₆, alcoxy en C₁ à C₆ et nitro,
avec, dans le cas où R³ est choisi parmi les radicaux alkyle en C₁ à C₆, alcoxy en C₁ à C₆, aryle en C₆ à C₁₀ et aryloxy en C₆ à C₁₀, un composé de formule (12) 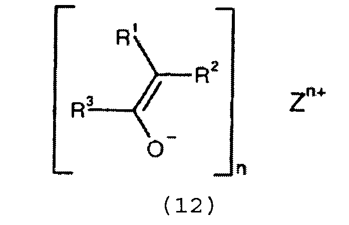
dans laquelle R¹ et R² sont tels que définis ci-dessus, Z est un cation, et n vaut 1, 2, 3 ou 4 ; ou
dans le cas où R³ est un radical hydroxy, un composé de formule (12a) 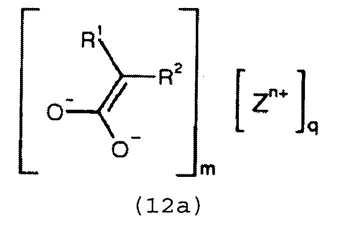
dans laquelle R¹ et R² sont tels que définis ci-dessus, et m, n et q sont des entiers positifs tels que nxq = 2m.

2. Procédé selon la revendication 1, dans lequel R¹ et R² sont tous deux méthyle.

3. Procédé selon la revendication 1 ou 2, dans lequel R³ est un radical alcoxy en C₁ à C₆.

4. Procédé selon la revendication 3, dans lequel R³ est méthoxy ou éthoxy.

5. Procédé selon la revendication 1, dans lequel X et Y sont tous deux trifluorométhanesulfonate ou tosylate.

6. Procédé selon l'une quelconque des revendications précédentes, dans lequel Zⁿ⁺ est Li⁺.

7. Procédé selon l'une quelconque des revendications précédentes, comprenant l'étape supplémentaire consistant à convertir le composé de formule (10) dans lequel R³ est choisi parmi les radicaux alcoxy en C₁ à C₆ en un bis(triméthylsilyloxy)cyclohex-1-ène de formule (18) 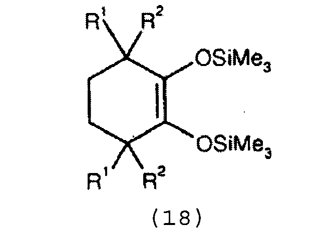
dans laquelle R¹ et R² sont tels que définis dans la revendication 1.

8. Procédé selon l'une quelconque des revendications précédentes, comprenant la ou les étapes supplémentaires consistant à convertir le composé de formule (10) ou (18) en cyclohexane-1,2-dione (17) 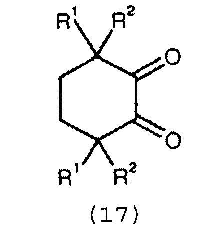
dans laquelle R¹ et R² sont tels que définis dans la revendication 1.

9. Procédé selon l'une quelconque des revendications précédentes, comprenant la ou les étapes supplémentaires consistant à convertir le composé de formule (10), (17) ou (18) pour former un composé de formule (20), (21), (24) ou (26) 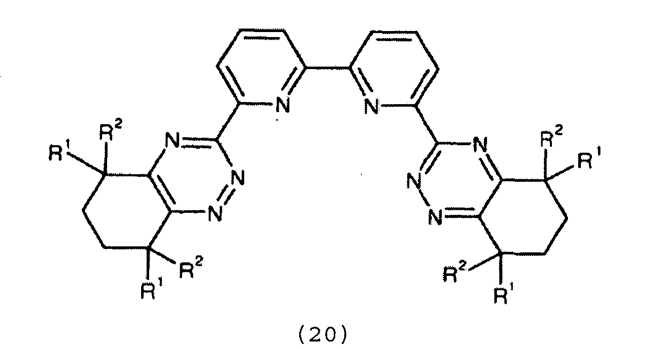 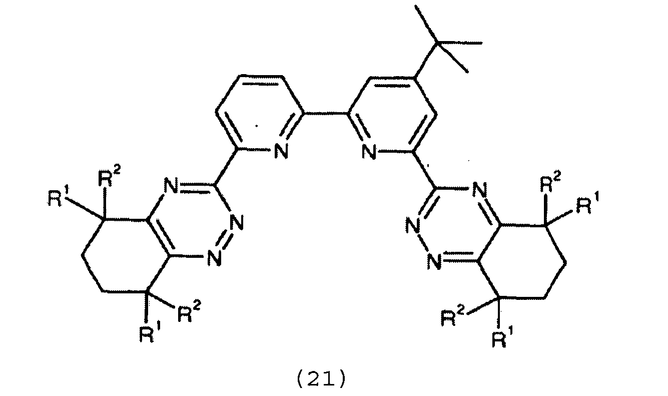 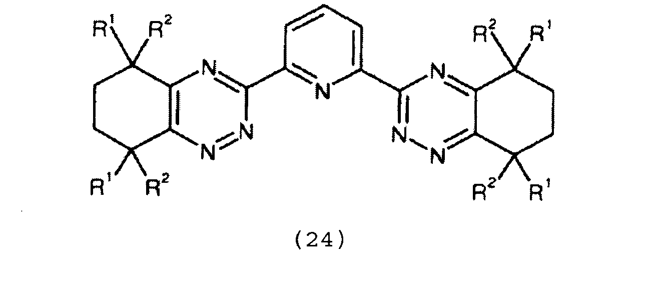 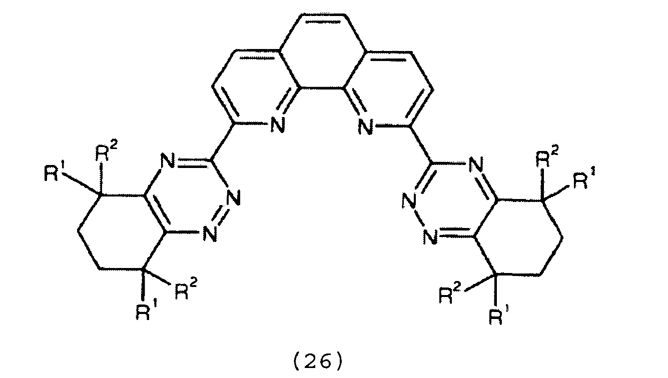
où R¹ et R² sont tels que définis dans la revendication 1.

10. Procédé selon l'une quelconque des revendications précédentes, comprenant la ou les étapes supplémentaires consistant à convertir le composé de formule (10), (17) ou (18) en un composé de formule (25) 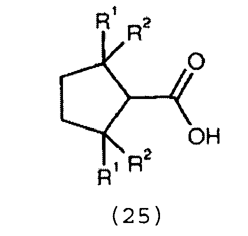
dans laquelle R¹ et R² sont tels que définis dans la revendication 1.
